# EUROPEAN PATENT SPECIFICATION

(11) **EP 1 377 677 B1**
(45) Date of publication and mention of the grant of the patent: **01.02.2012**
(21) Application number: 02733882.1
(22) Date of filing: 22.03.2002
(51) Int. Cl.: C12Q 1/00

(54) **COMPOSITION AND METHOD FOR DESTRUCTION OF INFECTIOUS PRION PROTEINS**
ZUSAMMENSETZUNG UND VERFAHREN ZUR ZERSTÖRUNG INFEKTIÖSER PRIONPROTEINE
COMPOSITION ET METHODE DE DESTRUCTION DES PROTEINES PRION INFECTIEUSES

(30) Priority: 12.04.2001 US 834284; 26.10.2001 US 7613
(43) Date of publication of application: 07.01.2004
(73) Proprietor: Bioresource International, Inc., Raleigh, NC 27606 (US)
(72) Inventor: SHIH, Jason, C., H., Cary, NC 27511 (US)
(74) Representative: Gee, Steven William
(86) International application number: PCT/US2002/008982
(87) International publication number: WO 2002/083082

(56) References cited:
- WO-A-00/65344
- US-A- 5 171 682
- US-A- 6 150 172
- US-B1- 6 214 565
- MCKINLEY M P ET AL: "A protease-resistant protein is a structural component of the scrapie prion" CELL, CELL PRESS, CAMBRIDGE, NA, US, vol. 35, November 1983 (1983-11), pages 57-62, XP002961643 ISSN: 0092-8674
- PERERA W S S ET AL: "Proteolytic fragmentation of the murine prion protein: role of Tyr-128 and His-177" FEBS LETTERS, ELSEVIER, AMSTERDAM, NL, vol. 463, no. 3, 17 December 1999 (1999-12-17), pages 273-276, XP004260801 ISSN: 0014-5793
- MARSH R F ET AL: "Physical and chemical properties of the transmissible mink encephalopathy agent" JOURNAL OF VIROLOGY, NEW YORK, US, US, vol. 3, no. 2, February 1969 (1969-02), pages 176-180, XP002961646 ISSN: 0022-538X
- CHO H J: "Inactivation of the scrapie agent by pronase" CANADIAN JOURNAL OF COMPARATIVE MEDICINE - REVUE CANADIENNE DE MEDECINE COMPAREE, OTTAWA, CA, vol. 47, 1983, pages 494-496, XP002961645 ISSN: 0008-4050
- SCHALLER O ET AL: "VALIDATION OF A WESTERN IMMUNOBLOTTING PROCEDURE FOR BOVINE PRPSC DETECTION AND ITS USE AS A RAPID SURVEILLANCE METHOD FOR THE DIAGNOSIS OF BOVINE SPONGIFORM ENCEPHALOPATHY (BSE)" BIOSIS, vol. 98, no. 5, 1999, pages 437-443, XP002158738
- BOLTON D C ET AL: "Molecular characteristics of the major scrapie prion protein" BIOCHEMISTRY, AMERICAN CHEMICAL SOCIETY. EASTON, PA, US, vol. 23, 1984, pages 5898-5906, XP002961644 ISSN: 0006-2960
- HUNTER G D ET AL: "Attempts to release the scrapie from tissue debris" JOURNAL OF COMPARATIVE PATHOLOGY, ACADEMIC PRESS, LONDON, GB, vol. 77, 1967, pages 301-307, XP002962234 ISSN: 0021-9975
- TAYLOR D M: "Inactivation of prions by physical and chemical means" JOURNAL OF HOSPITAL INFECTION, ACADEMIC PRESS, LONDON, GB, vol. 43, no. SUPPL, 1999, pages S69-S76, XP002977367 ISSN: 0195-6701
- MARSH ET AL.: 'Physical and chemical properties of the transmissible mink encephalopathy agent' JOURNAL OF VIROLOGY vol. 3, no. 2, 1969, pages 176 - 180, XP002961646
- CHO H.J.: 'Inactivation of the scrapie agent by pronase' CANADIAN JOURNAL OF COMPARATIVE MEDICINE vol. 47, 1983, pages 494 - 496, XP002961645
- SCHALLER ET AL.: 'Validation of a western immunoblotting procedure for bovine PrP(Sc) detection and its use as a rapid surveillance method for the diagnosis of bovine spongiform encephalopathy (BSE)' ACTA NEUROPATHOLOGY vol. 98, no. 5, November 1999, pages 437 - 443, XP002158738
- BOLTON ET AL.: 'Molecular characteristics of the major scrapie prion protein' BIOCHEMISTRY vol. 23, 1984, pages 5898 - 5906, XP002961644
- HUNTER ET AL.: 'Attempts to release the scrapie from tissue debris' COMPARATIVE PATHOLOGY vol. 77, 1967, pages 301 - 307, XP002962234
- MCKINLEY ET AL.: 'A protease-resistant protein is a structural component of the scrapie prion' CELL vol. 35, November 1983, pages 57 - 62, XP002961643
- 'WHO infection control guidlines for transmissible spongiform encephalopathies' WORLD HEALTH ORGANIZATION March 1999, pages 1 - 38, XP002961647
- RUTALA ET AL.: 'Creutzfeld-Jakob disease: recommendation for disinfection and sterilization' CLINICAL INFECTIOUS DISEASES vol. 32, 01 May 2001, pages 1348 - 1356, XP008012867

## Description

The present invention generally relates to a composition and method for destruction of infectious prion proteins associated with transmissible spongiform encephalopathy (TSE), e.g., bovine spongiform encephalopathy (BSE) and sheep scrapie. More specifically, the invention relates to application of Bacillus licheniformis PWD-1 keratinase for destroying infectious prion proteins contained in in vitro/ex vivo animal tissues, and/or for disinfecting and sterilizing medical devices and like articles contaminated by such infectious prior proteins.

### Background of the Invention

Prion proteins are conformationally anomalous proteins that are associated with infectious neurodegenerative diseases in human as well as non-human mammalian species.

Prion diseases in non-human mammalian species include scrapie (sheep), transmissible mink encepohalopathy (minks), chronic wasting disease (elk, deer), bovine spongiform encephalopathy (BSE) (cows), feline spongiform encephalopathy (cats), and simian spongiform encephalopathy (monkeys).

In humans a variety of neurodegenerative conditions are etiologically associated with prion proteins, including Creutzfeldt-Jakob disease, Gerstmann-Straussler-Scheinker syndrome, fatal insomnia, kuru, and variant Creutzfeldt-Jakob disease. Pathogenesis of human prion diseases is associated with carnivorism (BSE-infected beef, causing new variant Creutzfeldt-Jakob disease), administration of human growth hormone (causing iatrogenic Creutzfeldt-Jakob disease) and ritualistic cannibalism (causing kuru).

Over 180,000 BSE cases and 100 human Creutzfeldt-Jakob disease cases have been reported in Europe since 1992, and the human cases are predicted to significantly rise. The spread of such disease is difficult to contain, since such disease has no cure and the pathogenic prion protein is recalcitrant and non-immunogenic. The pathogenic and infectious isoform of prion protein is very stable, rich in β-sheet structure, and resistant to heat and common proteolytic enzymes (Prusiner, S.B., Proc. Natl. Acad. Sci. U.S.A., 95, 11363 (1998); Cohen, F.E. and Prusiner, S.B., Ann. Rev. Biochem., 67, 793 (1998); and Pan, K-M, Baldwin, M., Nguyen, J., Gasset, M., Serban, A., Groth, D., Mehlhom, I., Huang, Z., Fletterick, R.J., Cohen, F.E., and Prusiner, S.B., Proc. Natl. Acad. Sci. U.S.A, 90, 10962 (1993)).

Significant efforts have been focused on studies of BSE and prion protein contamination of human food supplies deriving from bovine sources, and of prion protein disease generation and propagation in bovine species. Infection in bovine populations has been associated with feeding of bovine herds with feedstocks containing bone meal and rendered organs and tissue deriving from infected cows, sheep and other ruminant animals.

At present, in many countries, animal products that otherwise would provide human food, and animal by-product that otherwise would provide a viable source of raw materials and nutritional supplements for animal feeds, are being incinerated and the ash residue buried, to preclude transmission of prion protein infection deriving from the presence of infectious prion proteins in associated animals.

In Europe, meat bone meal from animal by-products has been banned for feed use. In the United States, no outbreak of BSE has been reported, however, animal and rendering industries have been placed under restrictive regulations to prevent the incidence and spread of disease (Franco, B.A., Feed Stuffs, February 12, 2001). Further, the United States has banned imports of meat and meat by-products.

A variety of tests for determining the presence of infectious prion proteins in animal tissue have been developed, including Western blot tests, sandwich immunoassay tests, ELISA tests, fluoroimmunoassay tests, capillary immuno-electrophoresis tests, and plasminogen binding tests (Genetic Engineering News, Vol. 21, No. 6, March 15, 2001), but corresponding capability for industrially-applicable removal of infectious prion proteins from infected animal tissue has not evolved to date.

Infectious prion proteins are resistant to destruction by conventional methods that denature and otherwise degrade conformationally normal proteins, including methods such as autoclaving (even temperatures as high as 200°C are not effective to inactivate infectious prion proteins), boiling, freezing, and exposure to reagents such as formaldehyde, carbolic acid and chloroform. Typically, incineration or treatment with bleach is employed to destroy the pathogenic isoform of the prion protein.

It therefore would be a significant advance in the art to provide a composition and methodology for destruction of infectious prion proteins, which is applicable to the treatment of biological materials, e.g., animal tissue containing or contaminated with infectious prion proteins.

Moreover, the cross-contamination caused by reuse of medical instruments that have been previously exposed to prion-infected tissues is becoming an increased hazard and potential contributor to the transmission of infection.

The use of antiseptics, disinfectants, and sterilization procedures in health care facilities is critical to prevent the cross-contamination by medical instruments used during health care procedures. Disinfection and sterilization of medical devices or instruments are achieved by a variety of conventional methods, using various physical and chemical processes that destroy infectious biological materials, such as bacteria or viruses. For example, chemical disinfectants such as peracetic acid, hydrogen peroxide, sodium hydroxide, formic acid, bleach, alcohols, ethylene oxide, formaldehyde, formalin, and glutaraldehyde can be used for disinfecting and sterilizing medical devices; incineration, autoclaving, freezing, dry heating, boiling, UV and microwave radiation are also useful for destroying traditional infectious agents such as bacteria and viruses.

However, as discussed hereinabove, infectious prion proteins are known to be resistant to destruction by the conventional methods, which are therefore ineffective for disinfecting or sterilizing prion-contaminated medical devices or similar articles.

It is therefore an object of the present invention to provide a composition and methodology for effectively disinfecting or sterilizing prion-infected medical devices such as surgical instruments, or like articles such as kitchen utensils and laboratory tools.

### SUMMARY OF THE INVENTION

The invention provides a method and composition for destruction of infectious prion proteins.

In one aspect, the present invention relates to a method of treatment for reduction of infective prion protein at a locus that comprises, or is contaminated or suspected of being contaminated with, in vitro/ex vivo animal tissue containing infective prion protein, the method comprising the steps of:
(a) heating the locus to a sufficient temperature and for sufficient time to enhance the proteolytic susceptibility of infective prion protein at the locus; and
(b) exposing the heated locus to a *Bacillus licheniformis* PWD-1 keratinase enzyme that is effective for at least partial reduction of the infective protein prion at such locus, wherein the step of exposing the heated locus is conducted at a temperature that is lower than the temperature to which the locus is heated in step (a).

Said locus can be either tissue containing or contaminated by infective prion protein therein, or article(s) that are susceptible to contamination by infectious prion protein.

The temperature in step (a) does not exceed about 150°C, and preferably within a range of from about 100°C to about 150°C, more preferably within a range of from about 125°C to about 140°C.

Step (b) is carried out, for example, within a range of: (1) from about 35°C to about 100°C, (2) from about 40°C to abut 75°C, and (3) from about 50°C to about 60°C. Preferably, step (b) is carried out at a temperature above about 40°C, or more preferably, above about 50°C.

Keratinases are a group of proteolytic enzymes that are generally known as being capable of breaking down keratin proteins that are the major components of feather, horn, hooves, and hair. The inventor of the present application has discovered an unexpected and surprising result that keratinase enzymes are also effective in destroying infectious prion proteins, especially if the infectious prion proteins have been rendered proteolyticly susceptible. Moreover, such method as described hereinabove may further comprise a step of (c) testing the locus to verify reduction of infective prion protein therein, which comprises subject the locus to a test selected from the group consisting of Western blot tests, sandwich immunoassay tests, ELISA tests, fluoroimmunoassay tests, capillary immuno-electrophoresis tests, and plasminogen binding tests. Western blot test is preferred in practicing the present invention.

One specific aspect of the present invention relates to a method of treatment for reduction of infective prion protein at a locus contaminated or suspected of being contaminated with infective prion protein, as described hereinabove, wherein such locus comprises animal tissue containing or contaminated by infective prion protein therein, and wherein said tissue locus does not comprise tissue in a living human or animal. Such tissue comprises preferably mammalian tissue such as bovine tissue, ovine tissue, etc., and it can be from any body parts of the animal, such as brain, pituitary, intestine, lung, heart, kidney, and spleen tissues. Preferably, such animal tissue comprises nervous system tissue, and more preferably comprises BSE-infected or scrapie-infected tissue. It can be obtained from a carrier animal for the infective prion protein.

Another specific aspect of the present invention relates to a method of treatment for reduction of infective prion protein at a locus contaminated or suspected of being contaminated with infective prion protein, as described hereinabove, wherein such locus comprises article(s) susceptible to contamination by infectious prion protein. Such article(s) may comprise surgical instrument(s), such as clamps, forceps, scissors, knives, cables, punches, tweezers, cannulae, calipers, carvers, curettes, scalers, dilators, clip applicators, retractors, contractors, excavators, needle holders, suction tubes, coagulation electrodes, electroencephalographic depth electrodes, rib and sternum spreaders, bipolar probes, and rib shears. Alternatively, such article(s) may comprises cutleries and kitchen utensils, such as knives, forks, scissors, peelers, parers, slicers, spatulas, and cleavers, or laboratory apparatus(es), such as containers, filtration devices, centrifuges, spectrophotometers, and fluorometers, or veterinary devices, such as clamps, forceps, knives, saws, probes, and electronic stun equipment.

When the locus to be treated comprises article(s), the proteolytic enzyme used in step (b) is preferably provided in a solution form for purpose of cleansing and sterilizing such articles. Such enzyme solution is preferably characterized by a low effective concentration within the range of from about 0.2 g/L to about 1.0 g/L.

A further preferred aspect of the present invention relates to a method as herein defined for enhancing degradability of an infectious prion protein by keratinase enzymatic degradation treatment with a *Bacillus licheniformis* PWD-1 keratinase enzyme, including (a) heating the contaminated tissue or article to a first elevated temperature below the pyrolytic destruction temperature of the prion protein, followed by (b) enzymatic degradation treatment of the contaminated tissue or article at a second elevated temperature that is lower than the first elevated temperature, for at least partial reduction of the infectious prion protein that contaminates said tissue or article.

Other aspects, features and embodiments of the invention will be more fully apparent from the ensuing disclosure and appended claims.

### BRIEF DESCRIPTION OF THE DRAWING

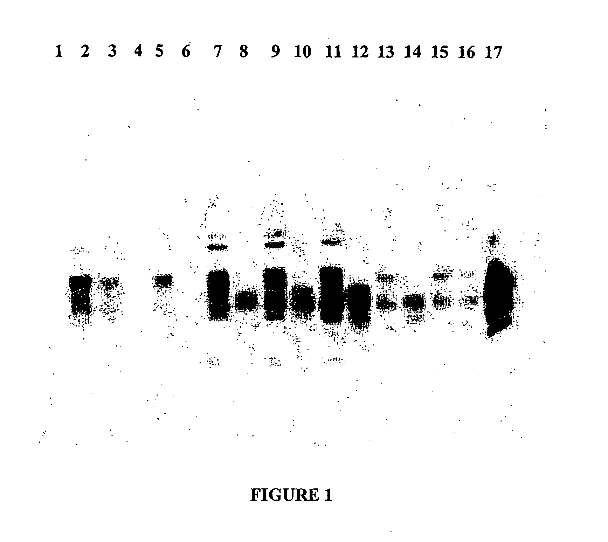

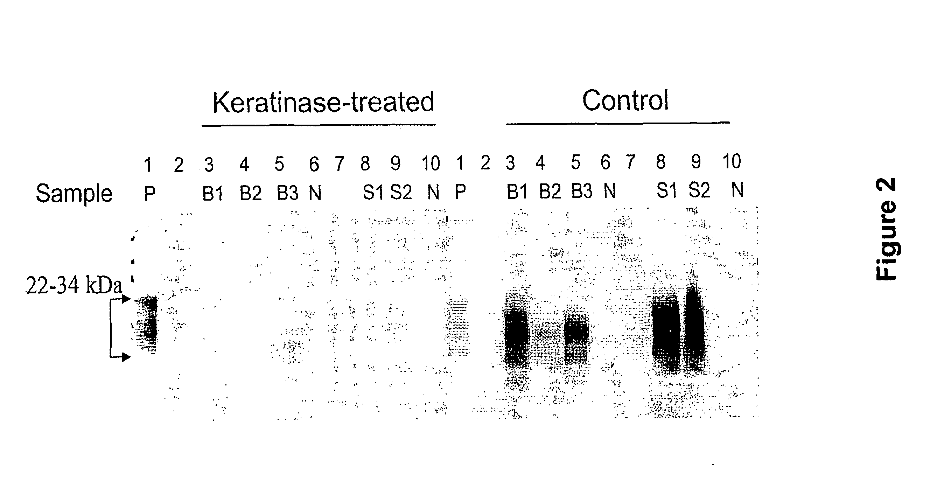

Figures 1 and 2 illustrate gel electrophoresis/Western blot results on SDS-PAGE gel, evidencing the efficacy of the method of the invention for destruction of infectious prion protein.

### DETAILED DESCRIPTION AND PREFERRED EMBODIMENTS OF THE INVENTION

Relative to the present invention and its features, aspects and embodiments as more fully described hereinafter, the disclosures of the technical literature cited in the Background of the Invention section hereof, as well as the following patents and technical literature articles;

U.S. Patent Nos.: 4,908,220; 4,959,311; 5,063,161; 5,171,682; 5,186,961; and 5,712,147;
Deslys, J.P., "Screening slaughtered cattle for BSE," Nature, Vol. 409, pp. 476-477, January 25, 2001; and

Cohen, F.E., "Protein Misfolding and Prion Diseases," J. Mol. Biol. (1999), Vol. 293, pp. 313-320.

The present invention is based on the use of keratinase enzymes for degradation of infectious prion protein in tissue, and alternatively, for disinfection or sterilization of prion-contaminated articles such as surgical instruments, cutleries and kitchen utensils, veterinary tools, and laboratory tools.

The efficacy of the process of the present invention for degradation of the infectious prion protein is wholly unexpected since high temperature exposure (e.g., at 200°C) of infectious prion proteins alone does not alter their pathogenic character; additionally, conventional proteolytic enzymes such as proteinase K that fully digest non-infectious PrP^{c} do not destroy the corresponding infectious isoform. It therefore is highly surprising that temperatures well below the incineration temperatures heretofore necessary for destruction of infectious PrP^{Sc} can be employed for enzymatic treatment, to totally eliminate infectious PrP^{Sc} from tissue containing or contaminated with same.

As used herein, the term elevated temperature means temperature of at least 35°C. The term proteolytic susceptibility means the ability of an infective prion protein to be enzymatically degraded to a non-infective product.

The treatment of a locus, such as tissues or articles, for reduction of infective prion protein associated therewith, can be carried out by various techniques as hereinafter described.

For example, the tissue or the article (which may contain, be contaminated with, or be suspected to contain or be contaminated with, infectious prion protein) in one embodiment of the invention is heated to a sufficient temperature and for sufficient time to enhance the proteolytic susceptibility of infective prion protein that may be present, in conjunction with exposure of the tissue or article to a keratinase enzyme that is effective to at least partially destroy any infective prion protein that is present.

Such treatment can be carried out in a two-step sequence, including an initial step of heating the tissue or article to a first higher elevated temperature and then exposing the heated tissue or article to the enzymatic agent at a second lower elevated temperature, for proteolytic degradation of the infectious prion protein.

In such two-step process, the tissue or article can be thermally treated at a first higher elevated temperature, and then cooled to second lower elevated temperature, e.g., by radiative heat loss from the tissue or article, convective cooling of the tissue or article, or in other appropriate manner, so that the tissue or article is at a suitable temperature when it is inoculated with or otherwise exposed to the keratinase enzyme in the second enzymatic treatment step.

In the second step of such two-step process, the tissue or article is exposed to a keratinase enzyme that is effective to at least partially destroy the infective protein prion associated with the tissue or article.

The method can therefore be carried out in various embodiments in which proteolytic susceptibility of infective prion protein associated with the tissue or article is enhanced by heating of the tissue or article to an elevated temperature for subsequent proteolytic enzyme treatment. The elevated temperature in the heating step may be any suitable temperature, e.g., at least 35°C, at least 40°C, at least 60°C, at least 75°C, and/or no more than 150°C (or other lower temperature, as desired), with one illustrative specific temperature range being from about 100°C to about 150°C, and more preferably from about 125°C to about 140°C.

The enzymatic degradation step can be carried out at any suitable temperature in the practice of the invention, e.g., at a temperature above about 35°C, above about 40°C, or above about 50°C, depending on the thermostable character of the keratinase enzyme employed.

By way of illustrative examples, the enzymatic degradation step can be conducted at a temperature in a range of from about 35°C to about 100°C, from about 40°C to about 100°C, from about 50°C to about 100°C, from about 40°C to about 75°C, or from about 50°C to about 60°C, depending on the proteolytic stability and enzymatic activity of the keratinase enzyme that is employed.

In the enzymatic degradation step, the keratinase enzyme at least partially, and preferably completely, destroys the infective protein prion that is in or otherwise associated with the tissue or article to be treated.

Specific temperature treatment conditions for the enzymatic treatment, as well as the temperature conditions necessary or desirable for any elevated temperature initial treatment step(s) that precede such enzymatic treatment, can be readily empirically determined without undue experimentation, within the skill of the art.

The keratinase enzyme may be used in a purified and concentrated form, or alternatively in a diluted form. It is preferred that the enzyme is dissolved in a solvent to form an enzyme solution with a concentration of from about 0.2 g/L to about 1.0 g/L.

The effective concentration required for the keratinase enzyme solution is significantly lower than that of the conventional enzyme detergents or disinfectants. Moreover, keratinase enzymes are characterized by an optimal active temperature range of from about 50°C to about 65°C at pH value of from about 6.0 to about 9.5, which is significantly higher than that of most conventional enzyme detergents. Therefore, the cleansing temperature of the process of the present invention can be significantly increased, which is more efficient for enhancing the proteolytic susceptibility of the infective prion protein associated with the surgical instruments.

In the method of the invention for treating in vitro/ex vivo tissues, the tissue being treated may be of any suitable type, including mammalian as well as non-mammalian animal tissues, and even plant tissues that actually or potentially contain infectious prion proteins. Mammalian tissues can include human as well as non-human mammalian tissues.

In a specific aspect, the in vitro/ex vivo tissues treatable in the method of the invention include, without limitation, bovine tissue, ovine tissue, simian tissue, and human tissue, and include various tissue types, e.g., brain, pituitary, intestine, lung, heart, kidney, and/or spleen tissue. In one aspect, the method of the invention is employed to treat nervous system tissue, which may be central nervous system tissue and/or peripheral nervous system tissue.

The method of enzymatically removing infectious prion proteins from tissue or disinfecting/sterilizing article such as surgical instrument or the like, in accordance with the present invention, can further include the step(s) of testing the tissue or article to verify destruction of infective prion protein associated therewith, after keratinase enzymatic treatment has been concluded. The testing of the tissue or article for infectious prion protein may be carried out in any suitable manner and with any suitable testing technique or methodology, e.g., by subjecting the tissue or article to a Western blot test, sandwich immunoassay test, ELISA test, fluoroimmunoassay test, capillary immuno-electrophoresis test, plasminogen binding test, or other suitable test that is efficacious for determining the presence or absence of infectious prion protein in the tissue being treated.

The enzymatic treatment method of the invention can be carried out in any suitable manner, with any appropriate sequence of processing steps.

For example, in one embodiment, the tissue or article to be treated is subjected to initial non-enzymatic thermal treatment as necessary or desired, followed by enzymatic treatment for destruction of infective or contaminative prion protein, followed by rinsing and non-enzymatic treatment, testing of the tissue or article treated, and/or further thermal/enzymatic treatment (e.g., in an alternating and repetitive cycle of non-enzymatic thermal treatment, and enzymatic elevated temperature treatment), as required, if the posttreatment testing shows incomplete removal of the infectious prion protein from the tissue or article.

Further, the method of the invention may include an initial determination of the presence of infectious prion protein associated with the tissue or article being treated, before any thermal/enzymatic treatment, so that treatment is only applied to tissue or article that is established by such determination as containing infectious prion protein.

Alternatively, the thermal/enzymatic treatment may be administered to tissue or article that may potentially contain or be contaminated with, but is not definitively ascertained beforehand to verify the presence of, infective prion protein, followed by testing of the treatment product for determination of the presence or absence of any infectious prion protein associated therewith.

The method of the invention is efficaciously applied to destroy infectious prion proteins in meat and meat by-products that are susceptible to containing or being contaminated with infectious prion proteins mediating TSEs such as BSE.

As such, the method of the invention provides a reliable approach to the treatment of bovine and other animal products and by-products that then can be further processed in food processing and/or rendering operations, rather than being incinerated to avoid transmission of BSE and other infectious prion protein diseases.

In a specific illustrative embodiment of the invention, infective prion protein is removed from bovine tissue containing same, by cooking the bovine tissue at temperature in a range of from about 100°C to about 150°C, e.g., for a time of from about 5 minutes to about 5 hours, followed by exposing the bovine tissue to a keratinase enzyme at temperature in a range of from about 35°C to about 100°C at which the proteolytic enzyme is thermally stable and proteolytically effective to destroy the infective prion protein in the bovine tissue.

The cooking treatment can be carried out in a suitable chamber or vessel in which elevated temperature conditions are appropriately maintained, optionally with control of pressure to provide a desired atmospheric, sub-atmospheric, or superatmospheric pressure in the cooking operation.

After cooking, the bovine tissue is subjected to proteolytic enzymatic treatment with *Bacillus licheniformis* PWD-1 keratinase to destroy all infectious prion protein therein. Following the thermal/enzymatic treatment, the tissue may be processed in any suitable manner.

For example, such bovine tissue, e.g., after testing or assay verification of the complete destruction of infectious prion protein, can be processed to yield an animal feed ingredient (e.g., meat bone meal) or feed supplement.

In one particularly preferred process embodiment, the cooking step is carried out in a first elevated temperature range of from about 125°C to about 150°C, and enzymatic treatment of the bovine tissue thereafter is carried out using *Bacillus licheniformis* PWD-1 keratinase, in a second elevated temperature range of from about 40°C to about 60°C.

The method of the present invention enables the utilization of rendered bovine meat by-products that would otherwise (in suspicion or verification of the presence of infectious prion proteins), require incineration and disposal.

The inventive method thereby achieves a substantial advance in the art, permitting nutritional use of material that would otherwise, in the absence of treatment, constitute a biological hazard. The inventive method concurrently avoids the costs and infrastructure requirements for incineration and disposal of infected or contaminated animal tissue.

The invention embodies a simple methodology for removing infectious prion protein, e.g., BSE-mediating prion protein, from tissue, by exposing the tissue to a keratinase enzyme that is thermally stable, at sufficient temperature and for sufficient time to at least partially clear the infectious prion protein from the tissue.

The method of the invention is broadly applicable to the destruction of prion protein that is infectious for transmissible spongiform encephalopathy (TSE) and/or for other prion protein-mediated diseases, including, without limitation, bovine spongiform encephalopathy (BSE) and sheep scrapie.

The method of the invention has applicability to processing animal meat for human food and processing of animal by-product for animal feed or an animal feed ingredient.

The present invention in one compositional aspect comprehends a tissue composition comprising tissue, e.g., bovine tissue, containing or contaminated with an infectious prion protein, such as prion protein mediating BSE, and *Bacillus licheniformis* PWD-1 keratinase enzyme that is thermally stable in a temperature range of from about 35°C to about 100°C, wherein said tissue does not comprise tissue in a living human or animal.

Such tissue composition may be at elevated temperature. The composition is enzymatically reactive at suitable elevated temperature to produce a product composition including the keratinase enzyme and the treated tissue free of infectious prion protein.

Another important application of the method of the present invention is disinfection and/or sterilization of articles, such as surgical instruments, cutleries, kitchen utensils, laboratory apparatus, and veterinary tools. Such method is broadly applicable to the destruction of prion protein contaminates associated with:
(a) surgical instruments, such as clamps, forceps, scissors, knives, cables, punches, tweezers, cannulae, calipers, carvers, curettes, scalers, dilators, clip applicators, retractors, contractors, excavators, needle holders, suction tubes, trocars, coagulation electrodes, electroencephalographic depth electrodes, rib and sternum spreaders, bipolar probes, rib shears, etc.;
(b) cutlery and kitchen utensils, such as knives, forks, scissors, peelers, parers, slicers, spatulas, and cleavers; and
(c) laboratory tools, such as filtration devices, centrifuges, spectrophotometers, fluorometers, and various containers and
(d) veterinary tools and devices, such as clamps, forceps, knives, saws, probes and electronic stun equipment.

The above list is only illustrative of several applications of the present invention, and it should not be construed in any manner as to limit the scope of the present invention.

The following table shows a disinfection/sterilization cycle according to one embodiment of the present invention:

**TABLE I**

| **Steps** | **Temperature** | **Time** |
|---|---|---|
| Pre-Wash (cold water) | Room Temp. | 2-5 minutes |
| Heating | 35-100°C | 20-40 minutes |
| Cooling | 34-51°C | 2-10 minutes |
| Enzyme Wash | 34-51°C | 20-120 minutes |
| Sonication | 34-5 °C | 5 minutes |
| Detergent Wash | 51-57°C | 2-5 minutes |
| Rinse and Dry | Room Temp. | 5 minutes |
| Autoclave Sterilization | 200-500°C | - |

The features and advantages of the invention are more fully shown with reference to the following illustrative example.

### EXAMPLE 1

A feather-degrading bacterium, *Bacillus licheniformis* strain PWD-1, isolated from a thermophilic anaerobic digester for poultry waste (see C.M. Williams and J.C.H. Shih, J. Appl. Bacteriol. 67, 25 (1989); J.C.H. Shih, Poultry Sci. 72, 1617 (1993)) was the source of the keratinase enzyme (see X. Lin, C.G. Lee, E.S. Casale, and J.C.H. Shih, Appl. Environ. Microbiol., 58, 3271 (1992)) employed in this example.

The gene encoding this keratinase enzyme (see X. Lin, D.W. Kelemen, E.S. Miller and J.C.H. Shih, Appl. Env. Microbiol. 61, 1469 (1995)) has been isolated and sequenced, and scale-up fermentation production of this enzyme has also been accomplished (see J.J. Wang and J.C.H. Shih, J. Ind. Microb. Biotech. 22, 608 (1999)). This enzyme is a serine protease.

Crude and purified preparations of this keratinase were produced as previously described (see X. Lin, C.G. Lee, E.S. Casale, and J.C.H. Shih, Appl. Environ. Microbiol. 58, 3271 (1992) and J.J. Wang and J.C.H. Shih, J. Ind. Microb. Biotech. 22, 608 (1999)) and obtained from the Fermentation Facility at North Carolina State University, Raleigh, North Carolina (NCSU). The test for the effect of keratinase on PrP was carried out at Institute of Animal Science and Health at Lelystad (ID-Lelystad), The Netherlands.

Purified keratinase was compared with other proteases, including elastase, collagenase, proteinase K and trypsin (all from Sigma chemical Co.) in reacting with various kinds of substrates. Hydrolysis of keratin, elastin and collagen were measured by ninhydrin color reaction (A₄₅₀) of increased free amino groups (see X. Lin, C.G. Lee, E.S. Casale, and J.C.H. Shih, Appl. Environ. Microbiol. 58, 3271 (1992)). Free leucine was used as the standard to calculate the equivalent free amino groups. Casein hydrolysis was measured by the increased A₂₈₀ in the supernatant (see Price and Johnson, 1989). The results are presented in Table 1 below. For each given substrate, relative activities of all proteases were determined. Cumulative relative activity (CRA) demonstrated that the keratinase has a wide range of substrates and possesses high activity.

**TABLE 1. Relative specific activities of proteases against different substrates^{a}**

| Substrate | Keratinase | Elastase | Collagenase | Proteinase K | Trypsin |
|---|---|---|---|---|---|
| | | | | | |
| Keratin^{b} | **1.00** | 0.29 | 0.00 | 0.36 | 0.09 |
| Elastin^{b} | 2.52 | **1.00** | 0.43 | 0.57 | 0.61 |
| Collagen^{b} | 2.58 | 1.15 | **1.00** | 0.70 | 0.38 |
| Casein^{c} | 1.28 | 0.80 | 0.02 | **1.00** | 0.40 |
| | | | | | |
| CRA^{d} | 7.38 | 3.24 | 1.45 | 2.63 | 1.48 |

| | | | | | |
|---|---|---|---|---|---|
| ^{a}All enzyme activities were measured at their individual optimum conditions and compared. ^{b}Proteolysis measured by ninhydrin reaction (Lin et al., 1992). ^{c}Proteolysis measured by increased soluble A₂₈₀ (Price and Johnson, 1989). ^{d}Cumulative Relative Activity. | | | | | |

The test of the effect of keratinase on pathogenic PrP was carried out in an Isolation Facility in the Laboratory of Molecular Recognition, ID-Lclystad. The European Union-validated procedure of Prionics Check (Prionics AG, Zurich) was used to detect pathogenic PrP. The Prionics Check procedure is based on the Western blot technique and employs 6H4 monoclonal antibody to visualize the specific form of PrP (Prionics AG, Test for the Detection of BSE-prions in Cattle, Practical Product Information, Zurich (2000)).

In order to mimic a meat bone meal process, modifications were made from the original procedure. First, proteinase K in the standard procedure was replaced by keratinase. A crude preparation of keratinase was used. Second, the effect of a pre-cooking of the BSE tissue was tested. The homogenized tissue was cooked at 115°C for 40 minutes with a Vulcain pressure-cooker. Third, an anti-oxidant, sodium sulfite (Na₂SO₃), also was tested. The rest of the procedure was the same as described in the manufacturer's Practical Product Information (Prionics AG, Test for the Detection of BSE-prions in Cattle, Practical Product Information, Zurich (2000)).

The following protocol was employed. One g of BSE-positive brain tissue was mixed and homogenized with 9 ml of Prionics buffer. Half of the aliquot, 5 ml, was added with Na₂SO₃ to give a final concentration 0.1% and the other half, without Na₂SO₃. The aliquots were distributed 4 x 2.0 ml into autoclavable Falcon tubes. An additional 1.0 ml was for used for positive control, treated by the standard Prionics procedure, and another 1.0 ml, for no keratinase control. Two tubes, with and without Na₂SO₃, were pressure-cooked for 40 min and the other 2 tubes were not cooked. In the wells of the PCR plate, the samples, 150 µl each, were treated by keratinase (150 µg, 1,000 EU/mg) at 50°C for 0-time or 4 hrs. Keratinase was pre-dissolved in phosphate buffer, 0.05 M, pH 7.5. The reaction was stopped by the addition of Prionics Pefabloc, an inhibitor of serine protease. At the end of enzymatic incubation, 10 µl of each sample mixture was loaded onto SDS-PAGE gel, and the Prionics procedure of electrophoresis, Western blot, and immuno-chemiluminescence detection was followed.

The results of this experiment are shown in Figure 1 (effect of keratinase degradation on BSE-prion protein), wherein Lanes 1-17 are as follows:
Lane 1: buffer alone.
Lane 2: BSE-brain tissue as tested.
Lane 3: Pre-cooked with Na₂SO₃, keratinase stopped at 0-time.
Lane 4: Same as Lane 3, except keratinase digestion for 4 hr.
Lane 5: Pre-cooked without Na₂SO₃, keratinase stopped at 0-time.
Lane 6: Same as Lane 5, keratinase digestion for 4 hr.
Lane 7: Without pre-cooking, with Na₂SO₃, keratinase 0-time.
Lane 8: Same as Lane 7, except keratinase digestion 4 hr.
Lane 9: Without pre-cooking, without Na₂SO₃, keratinase 0-time.
Lane 10: Same as Lane 9, except keratinase digestion 4 hr.
Lane 11: Without pre-cooking, with Na₂SO₃, no keratinase.
Lane 12: Same as Lane 11, except incubation 4 hr (Note: keratinase was accidentally added).
Lane 13: Purified scrapie PrP with Na₂SO₃, keratinase stopped at 0-time.
Lane 14: Same as Lane 13, except keratinase digestion 4 hr.
Lane 15: Purified scrapie PrP with Na₂SO₃, without keratinase.
Lane 16: Same as Lane 15, except incubation 4 hr.
Lane 17: PrP standard.

As shown in Fig. 1, the digestive effect of keratinase on infectious PrP is evident, particularly when the samples were precooked at 115°C for 40 min (Lanes 3-6). Without precooking (Lanes 7-10), the keratinase was less effective, but keratinase still degraded more than half of the infectious PrP positive material. On purified sheep scrapie PrP, keratinase was found to be active as well (Lanes 13-16). The presence of Na₂SO₃ did not appear to make much difference (Lanes 15-16) either alone or with keratinase. The Lane 12 sample was accidentally added with keratinase and therefore was positive.

### EXAMPLE 2

Experiments have been conducted to further evidence the efficacy of the invention, according to the following experimental procedure:

PWD-1 keratinase, a serine protease derived from Bacillus licheniformis strain PWD-1, was used to effect degradation of PrP^{Sc} in infected bovine and ovine brain stem tissues. The Prionics Check test (Prionics AG, Zurich, Switzerland) was used to determine PrP^{Sc} in the samples, following precooking of the tissue sample, and digestion of the tissue sample with purified PWD-1 keratinase as the digestive enzyme.

In each instance, 1 g of BSE-positive brain stem tissue was mixed and homogenized with 9 ml. of Prionics homogenization buffer, followed by pressure-cooking at 115°C for 40 minutes with a Vulcan pressure cooker. Into a 96-well plate, 150 µl of each sample was added and treated with PWD-1 keratinase pre-dissolved in phosphate buffer, 0.05 M, pH 7.5. An enzyme concentration of 250 µg/ml was used. Enzymatic digestion was carried out at 50°C for 60 min. The reaction was stopped by the addition of 15 µl Pefabloc®, an inhibitor of serine protease. Time-zero samples were treated by first adding the inhibitor before the addition of the PWD-1 keratinase. At the end of the enzymatic incubation, 10 µl of each sample was loaded onto the SDS-PAGE gel. Electrophoresis, Western blotting and immuno-chemiluminescence detection were conducted as outlined in the Prionics Check kit.

Brain stem tissue samples, 3 BSE-positive (samples B1, B2, B3), 1 negative (bovine sample N), 2 scrapie positive (samples S1 and S2) and 1 negative (ovine sample N) were tested by the purified PWD-1 keratinase, against corresponding controls.

Figure 2 shows the results, with the left-hand panel ("Keratinase-treated) being the result of keratinase digestion, and the right-hand panel ("Control") being the result of the standard Prionic Check. The keratinase was able to hydrolyze all PrP^{Sc} tested, BSE (lanes 3-5) as well as scrapie (lanes 8 and 9).

Figure 2 hereof evidences the complete destruction of infectious prion protein in bovine tissue samples containing same (samples B1, B2, B3) and in ovine samples containing same (samples S1 and S2), when such samples were treated by combined thermal and enzymatic treatment in accordance with the Invention.

These results demonstrate the versatility of the keratinase in degrading all types of proteins tested. In the test of the efficacy of the keratinase on BSE PrP, the results were positive, especially when the BSE brain tissue samples were pre-cooked. This is the first experiment to demonstrate that pathogenic PrP is degradable by an enzyme.

Pressure cooking at temperatures on the order of 125°C is a routine step in processing animal by-products into meat bone meal. Post-cooking treatment with keratinase in accordance with the invention, for destruction of infectious PrP, provides an effective method to control the spread of BSE. The keratinase-treated meat bone meal is readily tested to verify the absence of PrP, so that the meat bone meal can be recycled for feed use.

The method of the invention thus provides a simple and useful enzymatic treatment for animal product and by-product processing.

While the invention has been described herein with reference to various illustrative features, aspects, and embodiments, it will be appreciated that the utility of the invention is not thus limited, but rather extends to and encompasses other variations, modifications and other embodiments, as will readily suggest themselves to those of ordinary skill in the art.

Accordingly, the invention is to be broadly interpreted and construed as including such other variations, modifications and other embodiments, within the scope of the invention as hereinafter claimed.

### INDUSTRIAL UTILITY

The method and composition of the present invention are useful for destruction of infectious prion proteins, and are applicable to the treatment of biological materials, e.g., animal tissue containing or contaminated with infectious prion proteins. The invention enables processing of biological materials, which would otherwise (in suspicion or verification of the presence of infectious prion proteins) require incineration and disposal, into useful and safe animal feeds or other nutritional end products. The method and composition of the present invention are also useful for disinfection and/or sterilization of articles, such as surgical instruments, cutleries, kitchen utensils, laboratory apparatus, and veterinary tools, and effectively prevent cross-contamination and propagation of infective prion protein caused by reuse of such articles.

## Claims

1. A method of treatment for reduction of infective prion protein at a locus that comprises, or is contaminated or suspected of being contaminated with, in vitro/ex vivo animal tissue containing infective prion protein, the method comprising the steps of:
(a) heating the locus to a sufficient temperature and for sufficient time to enhance the proteolytic susceptibility of infective prion protein at the locus; and
(b) exposing the heated locus to a *Bacillus licheniformis* PWD-1 keratinase enzyme that is effective for at least partial reduction of the infective protein prion at such locus , wherein the step of exposing the heated locus is conducted at a temperature that is lower than the temperature to which the locus is heated in step (a).

2. The method of claim 1, wherein the temperature in step (a) comprises a temperature not exceeding 150°C.

3. The method of claim 1, wherein the temperature in step (a) comprises a temperature in a range of from 100°C to 150°C.

4. The method of claim 1, wherein step (b) is carried out at a temperature in a range of from 35°C to 100°C.

5. The method of claim 1, wherein step (b) is carried out at a temperature in a range of from 50°C to 60°C.

6. The method of claim 1, further comprising a step of (c) testing the locus to verify reduction of infective prion protein therein, by using a test selected from the group consisting of Western blot tests, sandwich immunoassay tests, ELISA tests, fluoroimmunoassay tests, capillary immuno-electrophoresis tests, and plasminogen binding tests.

7. The method of claim 1, wherein said locus comprises tissue containing or contaminated by infective prion protein therein, wherein said tissue locus does not comprise tissue in a living human or animal.

8. The method of claim 8, wherein said tissue is selected from the group consisting of brain, pituitary, intestine, lung, heart, kidney, and spleen tissues.

9. The method of claim 1, wherein said locus comprises article(s) susceptible to contamination by infectious prion protein.

10. The method of claim 9, wherein said article(s) comprise surgical instrument(s).

11. The method of claim 10, wherein said surgical instrument(s) are selected from the group consisting of: clamps, forceps, scissors, knives, cables, punches, tweezers, cannulae, calipers, carvers, curettes, scalers, dilators, clip applicators, retractors, contractors, excavators, needle holders, suction tubes, coagulation electrodes, electroencephalographic depth electrodes, rib and sternum spreaders, bipolar probes, and rib shears.

12. The method of claim 9, wherein said article(s) comprise cutleries and kitchen utensils.

13. The method of claim 9, wherein said article(s) comprise laboratory apparatus(es).

14. The method of claim 9, wherein said article(s) comprise veterinary devices.

15. The method of claim 1, wherein step (a) is carried out at a temperature in a range of from about 100°C to about 150°C and for a duration of from about 5 minutes to about 5 hours.

16. The method of claim 1, for enhancing degradability of infectious prion protein that contaminates a tissue or article by keratinase enzymatic degradation treatment with a *Bacillus licheniformis* PWD-1 keratinase enzyme, the method including (a) heating the contaminated tissue or article to a first elevated temperature below the pyrolytic destruction temperature of the prion protein, followed by (b) enzymatic degradation treatment of the contaminated tissue or article at a second elevated temperature that is lower than the first elevated temperature, for at least partial reduction of the infectious prion protein that contaminates said tissue or article.

17. The method of claim 16, wherein the tissue or article is exposed to the *Bacillus licheniformis* PWD-1 keratinase enzyme at a temperature that is above 40°C but below the pyrolytic destruction temperature of the prion protein.

18. A tissue composition comprising tissue containing or contaminated with an infectious prion protein and *Bacillus licheniformis* PWD-1 keratinase enzyme that is thermally stable in a temperature range of from about 35°C to about 100°C, wherein said tissue does not comprise tissue in a living humane or animal.

## Patentansprüche

1. Behandlungsverfahren zur Reduzierung von infektiösem Prion-Protein an einem Locus, der tierisches In-vitro-/Ex-vivo-Gewebe, welches infektiöses Prion-Protein enthält, umfasst oder damit kontaminiert ist mit bzw. in Verdacht steht, damit kontaminiert zu sein, das Verfahren umfassend die folgenden Schritte:
(a) das Erhitzen des Locus auf eine ausreichende Temperatur und für eine ausreichende Zeitdauer, um die proteolytische Empfindlichkeit des infektiösen Prion-Proteins an dem Locus zu erhöhen; und
(b) das Aussetzen des erhitzten Locus einem *Bacillus licheniformis-*PWD-1-Keratinase-Enzym, das wirksam ist, um zumindest teilweise das infektiöse Prion-Protein an einem solchen Locus zu reduzieren, wobei der Schritt des Aussetzens des erhitzten Locus bei einer Temperatur durchgeführt wird, die geringer ist als die Temperatur, auf die der Locus in Schritt (a) erhitzt wird.

2. Verfahren gemäß Anspruch 1, wobei die Temperatur in Schritt (a) eine Temperatur umfasst, die 150 °C nicht übersteigt.

3. Verfahren gemäß Anspruch 1, wobei die Temperatur in Schritt (a) eine Temperatur in einem Bereich von 100 °C bis 150 °C umfasst.

4. Verfahren gemäß Anspruch 1, wobei Schritt (b) bei einer Temperatur in einem Bereich von 35 °C bis 100 °C durchgeführt wird.

5. Verfahren gemäß Anspruch 1, wobei Schritt (b) bei einer Temperatur in einem Bereich von 50 °C bis 60 °C durchgeführt wird.

6. Verfahren gemäß 1, des Weiteren umfassend einen Schritt (c) des Testens des Locus, um die Reduzierung des darin enthaltenen infektiösen Prion-Proteins nachzuweisen, durch Verwendung eines Tests, der aus der Gruppe ausgewählt ist, welche umfasst: Western-Blot-Tests, Sandwich-Immunoassay-Tests, ELISA-Tests, Fluoroimmunoassay-Tests, Kapillar-Immuno-Elektrophoresetests und Plasminogen-Bindungstests.

7. Verfahren gemäß Anspruch 1, wobei der Locus Gewebe umfasst, das infektiöses Prion-Protein enthält oder damit kontaminiert ist, wobei der Gewebe-Locus kein Gewebe in einem lebenden Mensch oder Tier umfasst.

8. Verfahren gemäß Anspruch 8, wobei das Gewebe aus der Gruppe ausgewählt ist, welche umfasst: Gehirn-, Hypophysen-, Darm-, Lungen-, Herz-, Nieren- und Milzgewebe.

9. Verfahren gemäß Anspruch 1, wobei der Locus einen oder mehrere Gegenstände umfasst, die empfindlich sind für die Kontaminierung mit infektiösem Prion-Protein.

10. Verfahren gemäß Anspruch 9, wobei es sich bei dem Gegenstand bzw. den Gegenständen um ein chirurgisches Instrument bzw. chirurgische Instrumente handelt.

11. Verfahren gemäß Anspruch 10, wobei das chirurgische Instrument bzw. die chirurgischen Instrumente aus der Gruppe ausgewählt sind, welche umfasst: Klemmen, Zangen, Scheren, Messer, Kabel, Stanzen, Pinzetten, Kanülen, Tastzirkel, Modelliermesser, Küretten, Zahnsteinentferner, Dilatatoren, Klammerapplikatoren, Retraktoren, Kontraktoren, Exkavatoren, Nadelhalter, Saugrohre, Koagulationselektroden, elektroenzephalographische Tiefenelektroden, Rippen- und Brustbeinspreizer, bipolare Sonden und Rippenscheren.

12. Verfahren gemäß Anspruch 9, wobei es sich bei dem Gegenstand bzw. den Gegenständen um Bestecke und Küchengeräte handelt.

13. Verfahren gemäß Anspruch 9, wobei es sich bei dem Gegenstand bzw. den Gegenständen um ein Laborgerät bzw. um Laborgeräte handelt.

14. Verfahren gemäß Anspruch 9, wobei es sich bei dem Gegenstand bzw. den Gegenständen um Veterinärgeräte handelt.

15. Verfahren gemäß Anspruch 1, wobei Schritt (a) bei einer Temperatur in einem Bereich von etwa 100 °C bis etwa 150 °C und für eine Zeitdauer von etwa 5 Minuten bis etwa 5 Stunden durchgeführt wird.

16. Verfahren gemäß Anspruch 1, zur Erhöhung der Abbaubarkeit von infektiösem Prion-Protein, das ein Gewebe oder einen Gegenstand kontaminiert, durch Keratinase-Enzym-Degradationsbehandlung mit einem *Bacillus licheniformis*-PWD-1 -Keratinase-Enzym, wobei das Verfahren einschließt (a) das Erhitzen des kontaminierten Gewebes oder Gegenstands auf eine erste erhöhte Temperatur unterhalb der pyrolytischen Zerstörungstemperatur des Prion-Proteins, gefolgt von (b) der Enzym-Degradationsbehandlung des kontaminierten Gewebes oder Gegenstands bei einer zweiten erhöhten Temperatur, die niedriger ist als die erste erhöhte Temperatur, zur mindestens teilweisen Reduzierung des infektiösen Prion-Proteins, welches das Gewebe oder den Gegenstand kontaminiert.

17. Verfahren gemäß Anspruch 16, wobei das Gewebe oder der Gegenstand dem *Bacillus licheniformis*-PWD-1-Keratinase-Enzym bei einer Temperatur ausgesetzt wird, die über 40 °C aber unter der pyrolytischen Zerstörungstemperatur des Prion-Proteins liegt.

18. Gewebezusammensetzung, umfassend Gewebe, welches ein infektiöses Prion-Protein enthält oder damit kontaminiert ist, sowie ein *Bacillus licheniformis*-PWD-1-Keratinase-Enzym, das in einem Temperaturbereich von etwa 35 °C bis etwa 100 °C thermisch stabil ist, wobei das Gewebe kein Gewebe in einem lebenden Mensch oder Tier umfasst.

## Revendications

1. Un procédé de traitement pour la réduction de la protéine prion infectieuse sur un locus qui comprend, ou est contaminé ou suspecté d'être contaminé avec, in vitro/ex vivo, un tissu animal contenant la protéine prion infectieuse, le procédé comprenant les opérations suivantes :
(a) le chauffage du locus à une température suffisante et pendant une durée suffisante pour améliorer la susceptibilité protéolytique de la protéine prion infectieuse sur le locus, et
(b) l'exposition du locus chauffé à une enzyme kératinase PWD-1 *Bacillus licheniformis* qui est efficace pour une réduction au moins partielle du prion de la protéine infectieuse sur ledit locus, où l'opération d'exposition du locus chauffé est réalisée à une température qui est inférieure àla température à laquelle le locus est chauffé à l'opération (a).

2. Le procédé selon la Revendication 1, où la température à l'opération (a) comprend une température ne dépassant pas 150°C.

3. Le procédé selon la Revendication 1, où la température à l'opération (a) comprend une température dans la plage allant de 100°C à 150°C.

4. Le procédé selon la Revendication 1, où l'opération (b) est effectuée à une température dans une plage allant de 35°C à 100°C.

5. Le procédé selon la Revendication 1, où l'opération (b) est effectuée à une température dans une plage allant de50°C à 60°C.

6. Le procédé selon la Revendication 1, comprenant en outre une opération de (c) test du locus destiné à vérifier la réduction de la protéine prion infectieuse dans celui-ci au moyen d'un test sélectionné dans le groupe se composant detests de buvardage de Western, tests de dosage immunologique en sandwich, tests ELISA, tests de dosage fluoro-immunologique, tests immunoélectrophorèses capillaires et tests de liaison plasminogène.

7. Le procédé selon la Revendication 1, où ledit locus comprend un tissu contenant ou contaminé par la protéine prion infectieuse, où ledit locus de tissu ne comprend pas de tissu dans un humain ou animal vivant.

8. Le procédé selon la Revendication 8, où ledit tissu est sélectionné dans le groupe se composant de tissus du cerveau, pituitaires, de l'intestin, du poumon, du coeur, du rein et de la rate.

9. Le procédé selon la Revendication 1, où ledit locus comprend des articles qui sont susceptibles de contamination par la protéine prion infectieuse.

10. Le procédé selon la Revendication 9, où les dits articles comprennent des instruments chirurgicaux.

11. Le procédé selon la Revendication 10, où les dits instruments chirurgicaux sont sélectionnés dans le groupe se composant de : pinces, forceps, ciseaux, couteaux, câbles, emporte-pièces, pinces de serrage, canules, pieds à coulisse, spatules, curettes, instruments à détartrer, dilatateurs, applicateurs à pince, rétracteurs, contracteurs, excavateurs, porte-aiguilles, tubes d'aspiration, électrodes de coagulation, électrodes en profondeur électroencéphalographiques, écarteurs de côtes et de sternum, sondes bipolaires et costotomes.

12. Le procédé selon la Revendication 9, où les dits articles comprennent de la coutellerie et des ustensiles de cuisine.

13. Le procédé selon la Revendication 9, où les dits articles comprennent des appareils de laboratoire.

14. Le procédé selon la Revendication 9, où les dits articles comprennent des dispositifs vétérinaires.

15. Le procédé selon la Revendication 1, où l'opération (a) est effectuée à une température dans la plage allant d'environ 100°C à environ 150°C et pendant une durée allant d'environ 5 minutes à environ 5 heures.

16. Le procédé selon la Revendication 1, destiné à améliorer la dégradabilité de la protéine prion infectieuse qui contamine un tissu ou un article par traitement de dégradation enzymatique kératinase avec une enzyme kératinase PWD-1 *Bacillus licheniformis,* le procédé comprenant (a)le chauffage du tissu ou de l'article contaminé à une première température élevée inférieure à la température de destruction pyrolytiquede la protéine prion, suivi par(b) un traitement de dégradation enzymatique du tissu ou de l'article contaminé à une deuxième température élevée qui est inférieure à la première température élevée, pour une réduction au moins partielle de la protéine prion infectieuse qui contamine ledit tissu ou article.

17. Le procédé selon la Revendication 16, où le tissu ou article est exposé à l'enzyme kératinase PWD-1 *Bacillus licheniformis* à une température qui est supérieure à 40°C mais inférieure à la température de destruction pyrolytique de la protéine prion.

18. Une composition tissulaire comprenant un tissu contenant ou contaminé avec une protéine prion infectieuse et un enzyme kératinase PWD-1 *Bacillus licheniformis* qui est thermiquement stable dans une plage de température allant d'environ 35°C à environ 100°C, où ledit tissu ne comprend pas de tissu dans un humain ou animal vivant.
